# EUROPEAN PATENT APPLICATION

(11) **EP 4 239 069 A1**
(43) Date of publication of application: **06.09.2023**
(21) Application number: 21885365.3
(22) Date of filing: 01.11.2021
(51) Int. Cl.: C12N 15/14, C07K 14/765, A61P 3/10, A61P 3/04, A61P 9/10, A61P 25/28, A61P 25/16

(54) **USE OF HUMAN SERUM ALBUMIN IN TREATMENT OF DISEASES**

(30) Priority: 30.10.2020 CN 202011200484
(71) Applicant: Shenzhen Protgen Ltd., Shenzhen, Guangdong 518109 (CN)
(72) Inventor: LUO, Yongzhang, Beijing 100084 (CN); FU, Yan, Beijing 100084 (CN); LIU, Hongyi, Beijing 100084 (CN); JU, Anji, Beijing 100084 (CN); TANG, Jiaze, Beijing 100084 (CN); JIANG, Yi, Beijing 100084 (CN); MA, Boyuan, Beijing 100084 (CN); JIANG, Xiaoqin, Beijing 100084 (CN); FENG, Yu, Beijing 100084 (CN); CHANG, Guodong, Beijing 100085 (CN); LI, Hui, Beijing 100085 (CN)
(74) Representative: Symbiosis IP Limited
(86) International application number: PCT/CN2021/127957
(87) International publication number: WO 2022/089639

(57) **Abstract**

Provided is the use of human serum albumin in the manufacturing of a drug for treating diabetes, obesity, atherosclerosis, Alzheimer's disease, Parkinson's disease and other diseases. In a preferred embodiment, the human serum albumin is the recombinantly prepared young and uninjured human serum albumin, and has achieved excellent effects in reducing the blood sugar level of a diabetic patient.

## Description

### CROSS-REFERENCE TO RELATED APPLICATIONS

The present application claims the priority of Chinese patent application no. CN202011200484.6 filed on October 30, 2020, which is incorporated herein by reference in its entirety.

### Field of the Invention

The present invention relates to the field of biopharmaceuticals, and more particularly, to the use of human serum albumin (HSA) in the treatment of diabetes mellitus, Alzheimer's disease and other diseases.

### Background of the Invention

Human Serum Albumin (HSA; CAS: 70024-90-7) is an important component in human plasma with a molecular weight of 66 kD. It has a concentration of 42 g/L in plasma, and accounts for 60% of the total plasma protein content. As an important carrier protein, HSA is responsible for transporting fatty acids, bile pigments, amino acids, steroid hormones, metal ions and many therapeutic molecules etc., while maintaining the normal blood osmotic pressure. Clinically, HSA can act as a plasma volume expander to address the blood loss due to surgery, accidents or massive hemorrhage.

In recent years, due to the shortage of blood resources and the risk of getting a disease via blood, recombinant human serum albumin (rHSA) is attracting increasing attentions. As the HSA is used at a high dose in clinic, which can be up to ten to several tens of grams per day, the need thereof is great in many applications.

### Summary of the Invention

The inventors surprisingly found that human serum albumin (HSA), especially young and undamaged recombinant human serum albumin (rHSA), shows a significant therapeutic effect on various diseases.

The present invention provides use of human serum albumin (HSA) in the manufacture of a medicament for treating a disease selected from the group of diabetes mellitus, obesity, atherosclerosis, Alzheimer's disease, and Parkinson's disease.

The present invention also provides a method of treating a disease, comprising administering to a subject a therapeutically effective amount of human serum albumin (HSA), wherein the disease is selected from the group consisting of diabetes mellitus, obesity, atherosclerosis, Alzheimer's disease, and Parkinson's disease.

The present invention also provides use of human serum albumin (HSA) in treating a disease selected from the group consisting of diabetes mellitus, obesity, atherosclerosis, Alzheimer's disease, and Parkinson's disease.

The present invention also provides use of a pharmaceutical composition comprising a human serum albumin (HSA) and a pharmaceutically acceptable carrier in treating a disease, wherein the disease is selected from the group consisting of diabetes mellitus, obesity, atherosclerosis, Alzheimer's disease, and Parkinson's disease.

In some embodiments, the present invention also provides a method of regulating the blood glucose level in a subject, as well as a method of regulating the free fatty acid (FFA) or the cholesterol level in the blood of a subject, comprising administering to the subject an effective amount of HSA. The subject can be a healthy subject or a subject suffering from a disease such as diabetes mellitus or atherosclerosis.

In some embodiments, the HSA is an HSA prepared from human blood.

In some embodiments, the HSA is a young and undamaged HSA.

Preferably, the young and undamaged HSA exhibits at least one, for example, two, three, or preferably four, of the following properties: (1) a higher ratio of free thiol in Cys-34 residue, (2) a lower level of advanced glycation end-product (AGE), (3) a lower level of carbonylation, and (4) a lower level of homocysteinylation, as compared to an endogenous HSA preparation prepared from the serum of a young individual.

Preferably, the young and undamaged HSA exhibits at least one, for example, two, three, or preferably four, of the following properties: (1) the ratio of free thiol in Cys-34 residue is greater than 50%, for example, 70%, especially 80%, preferably 90%, and more preferably greater than 95%, as determined by the Ellman's method; (2) the level of advanced glycation end-product (AGE) is lower than 60 µg/g protein, preferably lower than 40 µg/g protein, and more preferably lower than 30 µg/g protein, as determined by ELISA (CLOUD-CLONE Co., CEB353Ge); (3) the level of carbonyl is lower than 1.7 nmol/mg protein, and preferably lower than 1.5 nmol/mg protein, as determined with the Protein Carbonyl Content Assay Kit; and (4) the level of homocysteine is lower than 5 nmol/g protein, preferably lower than 3.5 nmol/g protein, and more preferably lower than 2 nmol/g protein, as determined by ELISA (Jianglai, JL10022).

In a preferred embodiment of the present invention, the ratio of free thiol in Cys-34 residue is greater than 80%, the level of AGE is lower than 30 µg/g protein, the level of carbonyl is lower than 1.5 nmol/mg protein, and the level of homocysteine is lower than 2 nmol/g protein.

Preferably, the HSA is produced recombinantly.

### Brief Description of the Drawings

Figure 1A shows the changes of blood glucose in mice with type II diabetes. Fasting glycemia: fasting blood glucose; Injection: one injection as indicated by one arrow; Saline: the control group with physiological saline; rMSA: the treatment group with recombinant mouse serum albumin; WT: the wild type mice; db: the mice that are type II diabetes model. The Saline WT and the rMSA WT groups have 5 mice per group, while the Saline db and the rMSA db groups have 7 mice per group. The error bar indicates SEM.
Figure 1B shows the results of HE staining of mouse pancreas sections. Beta cell failure: islet β cell failure; No beta cell failure: no islet β cell failure; Saline: the control group with physiological saline; rMSA: the treatment group with recombinant mouse serum albumin; db: type II diabetes model mice.
Figure 1C shows the percentage of mice that have islet β cell failure in two groups of mice. * refers to Fisher's exact test. Saline: the control group with physiological saline; rMSA: the treatment group with recombinant mouse serum albumin; db: type II diabetes model mice.
Figure 1D shows the results of HE staining of mouse pancreas sections as well as marking and segmenting of the images of these sections. H&E: HE staining; Segment: marking and segmenting; Saline: the control group with physiological saline; rMSA: the treatment group with recombinant mouse serum albumin.
Figure 1E shows the percentage of the islet area in the entire pancreas area for a mouse. Saline: the control group with physiological saline; rMSA: the treatment group with recombinant mouse serum albumin; WT: the wild type mice; db: type II diabetes model mice. The error bar indicates SEM.
Figure 1F shows the relative mouse islet size. Saline: the control group with physiological saline; rMSA: the treatment group with recombinant mouse serum albumin; WT: the wild type mice; db: type II diabetes model mice. The error bar indicates SEM.
Figure 1G shows the results of immunofluorescence and TUNEL staining of the sections of db mouse pancreases. Insulin: insulin; TUNEL: terminal deoxynucleotidyl transferase-mediated nick end labeling; DAPI: diaminophenylindole; Saline: the control group with physiological saline; rMSA: the treatment group with recombinant mouse serum albumin.
Figure 1H shows the percentage of TUNEL-positive cells in insulin-positive cells in the sections of db mouse pancreases. Saline: the control group with physiological saline; rMSA: the treatment group with recombinant mouse serum albumin; db: type II diabetes model mice. The error bar indicates SEM.
Figure 1I shows the results of immunofluorescence staining of the sections of db mouse pancreases for cleaved caspase-3 (CC3). Insulin: insulin; CC3: cleaved caspase-3; Merge: merged image comprising the results of Insulin (shown in red in the image), CC3 (shown in green in the image), and DAPI (diaminophenylindole; shown in blue in the image); Saline: the control group with physiological saline; rMSA: the treatment group with recombinant mouse serum albumin.
Figure 1J shows the percentag of CC3-positive cells in insulin-positive cells in the sections of db mouse pancreases. Saline: the control group with physiological saline; rMSA: the treatment group with recombinant mouse serum albumin. The error bar indicates SEM.
Figure 1K shows the results of the glucose tolerance test in mice. Blood glucose: blood glucose content; min: minutes; Saline: the control group with physiological saline; rMSA: the treatment group with recombinant mouse serum albumin; WT: the wild type mice; db: type II diabetes model mice. The error bar indicates SEM.
Figure 1L shows the area under the curve in Figure 4K. Saline: the control group with physiological saline; rMSA: the treatment group with recombinant mouse serum albumin; WT: the wild type mice; db: type II diabetes model mice. The error bar indicates SEM.
Figure 1M shows the percentage of fat mass in total body weight of the db mouse. Saline: the control group with physiological saline; rMSA: the treatment group with recombinant mouse serum albumin. The error bar indicates SEM.
Figure 4N shows the percentage of lean mass in total body weight of the db mouse. Saline: the control group with physiological saline; rMSA: the treatment group with recombinant mouse serum albumin. The error bar indicates SEM.

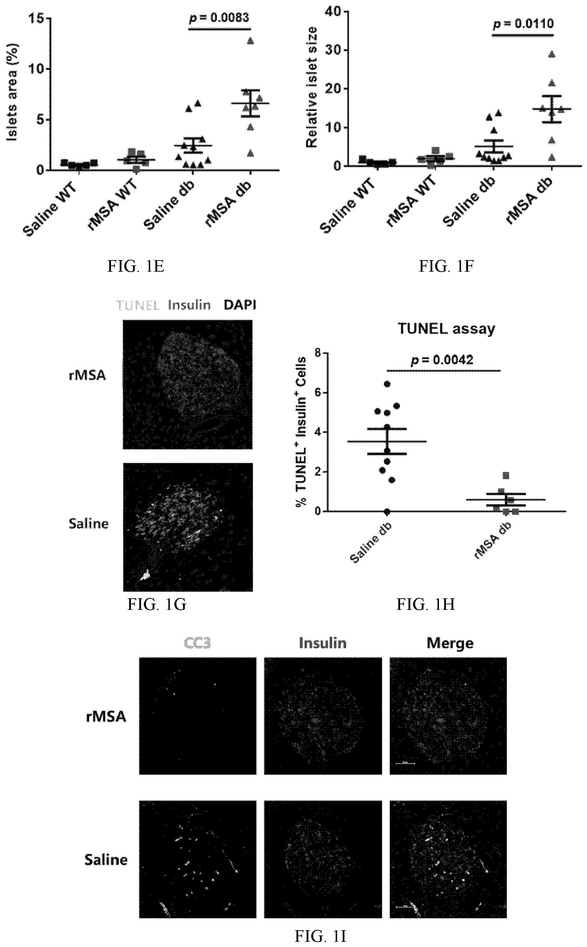

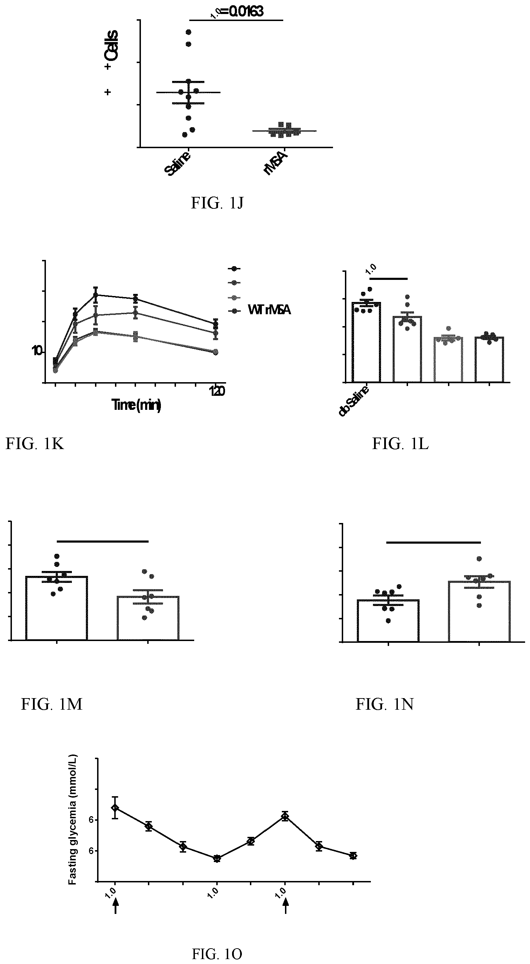

Figure 1O shows the changes of blood glucose in patients with type II diabetes. Fasting glycemi: fasting blood glucose; injection: one injection as indicated by one arrow. The error bar indicates SEM.

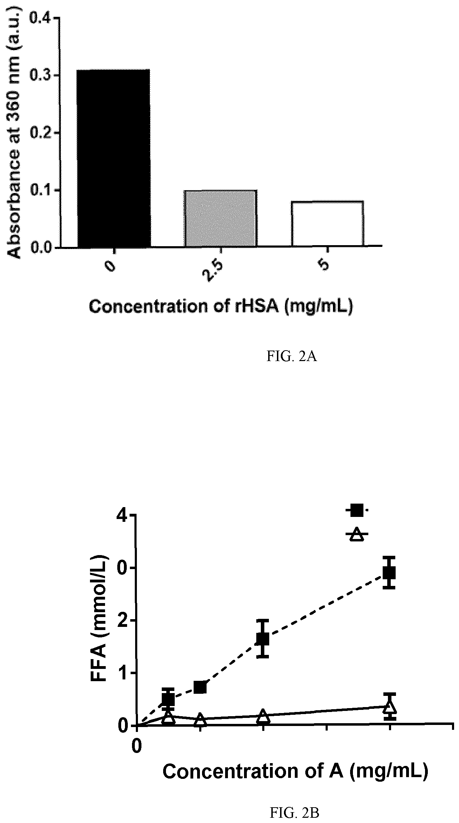

Figure 2A shows the ability of the rHSA (Protgen) in binding FFAs.
Figure 2B shows the comparison of the FFA contents of two HSA preparations. pHSA: the commercially available blood-derived HSA preparation; rHSA: the recombinant HSA preparation (Protgen). The error bar indicates SEM.

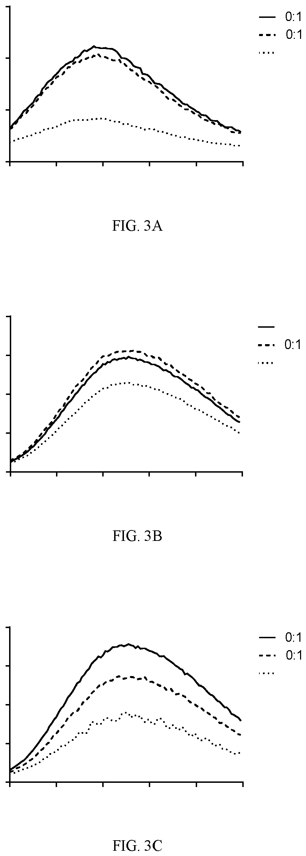

Figure 3A shows the fluorescence emission spectrum for the rHSA (Protgen) binding to cholesterol.
Figure 3B shows the fluorescence emission spectrum for the commercially available blood-derived HSA-1 binding to cholesterol.
Figure 3C shows the fluorescence emission spectrum for the commercially available blood-derived HSA-2 binding to cholesterol.

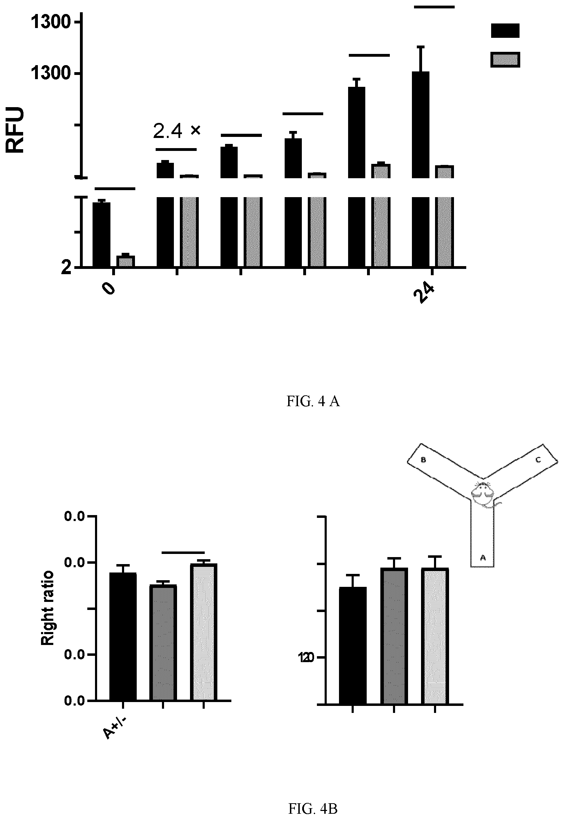

Figure 4A shows the ability of two HSA preparations in inhibiting the aggregation of Aβ peptides. RFU: relative fluorescence unit; pHSA: the blood-derived HSA preparation; rHSA: the recombinant HSA preparation (Protgen).
Figure 4B shows the performance of mice in the Y-maze. Right ratio: the correct rate; Total arm entries: the total number of explorations. The error bar indicates SEM.

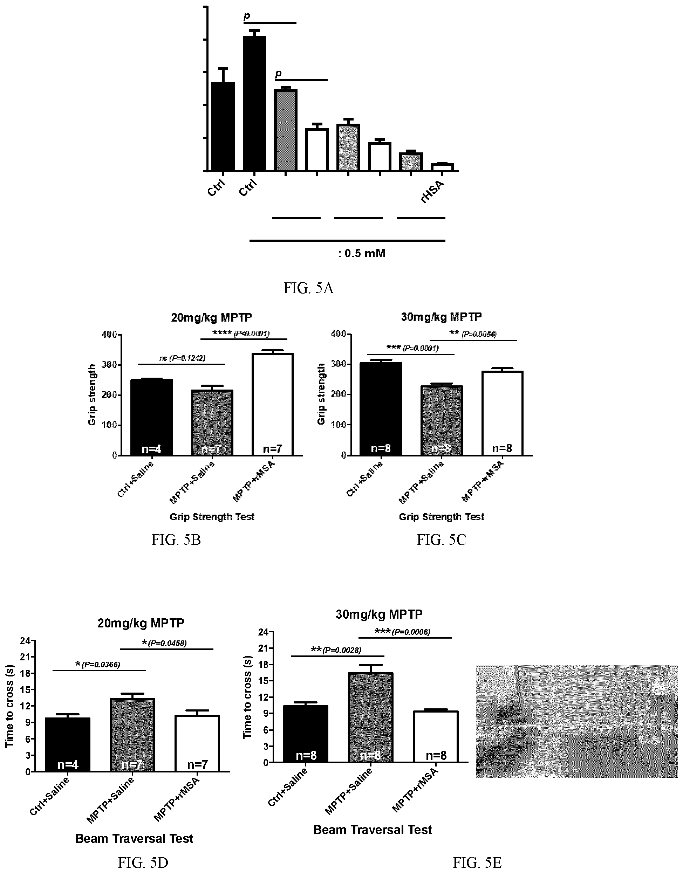

Figure 5A shows the effects of the young and undamaged rHSA and the commercially available blood-derived HSA on the ROS levels in SH-SY5Y cells induced with MPP+. pHSA: the commercially available blood-derived HSA preparation; rHSA: the recombinant HSA preparation (Protgen). The error bar indicates SEM.
Figure 5B shows that the rHSA increased the grip strength of mice having PD-like symptoms induced by a low dose of MPTP.
Figure 5C shows that the rHSA increased the grip strength of mice having PD-like symptoms induced by a high dose of MPTP.
Figures 5D and 5E show that the rHSA significantly shortened the time for the mice having PD-like symptoms induced by MPTP to cross a beam.

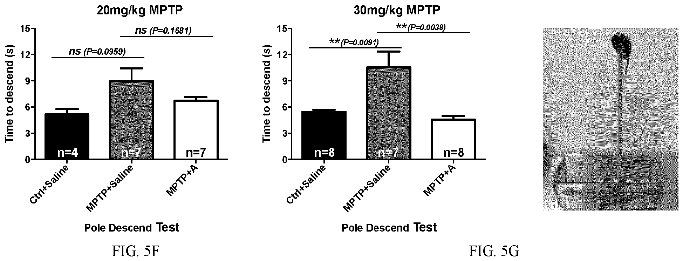

Figures 5F and 5G show that the rHSA significantly shortened the time for the mice having PD-like symptoms induced by MPTP to descend to the bottom of the pole.

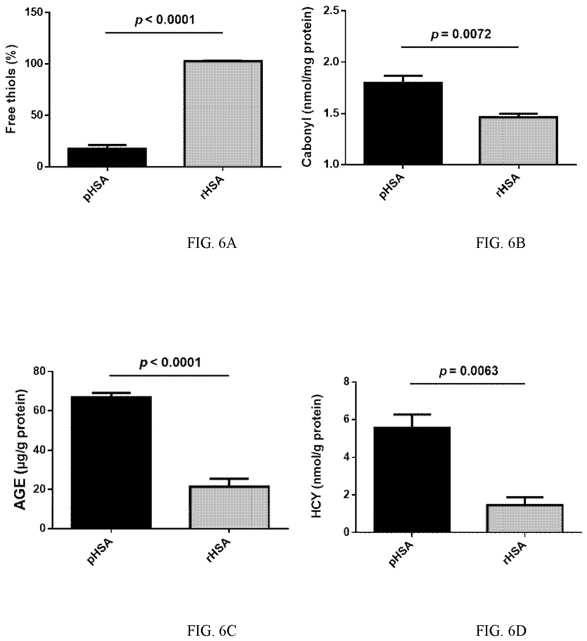

Figure 6 shows the comparison of four age-related indicators between the rHSA (Protgen) and the blood-derived HSA products from different brands. Free thiols: content of free thiol (FIG. 6A); Cabonyl: carbonyl content (FIG. 6B); AGE: level of advanced glycation end-product (FIG. 6C); HCY: content of homocysteine (FIG. 6D). The error bar indicates SEM.

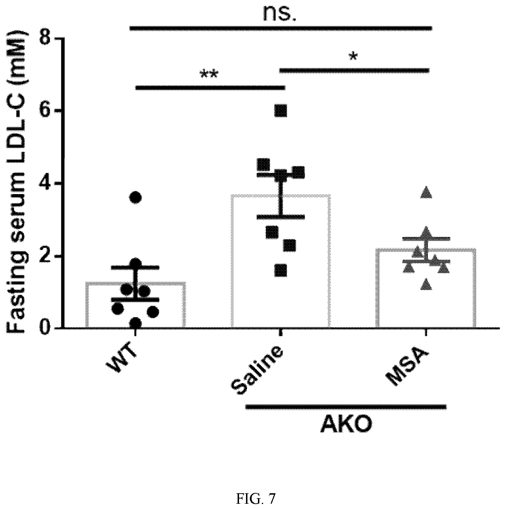

Figure 7 shows low-density lipoprotein cholesterol (LDL-C) levels in mice supplemented with the recombinant mouse serum albumin (rMSA) or physiological saline. AKO: mice with the albumin gene knocked out; WT: wild type mice; rMSA: recombinant mouse serum albumin. The error bar indicates SEM.

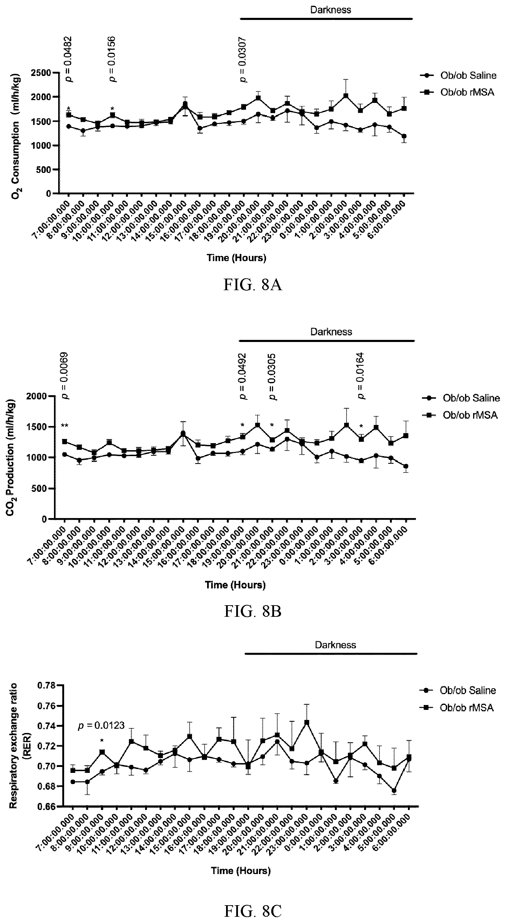

Figure 8A shows the oxygen consumption by obese mice. Saline: the control group with physiological saline; rMSA: the treatment group with recombinant mouse serum albumin; ob: obese mice as models. The Ob/ob Saline and Ob/ob rMSA groups have 4 mice per group. The error bar indicates SEM.
Figure 8B shows the carbon dioxide production by obese mice. The error bar indicates SEM.
Figure 8C shows the respiratory exchange ratio in mice. The error bar indicates SEM.

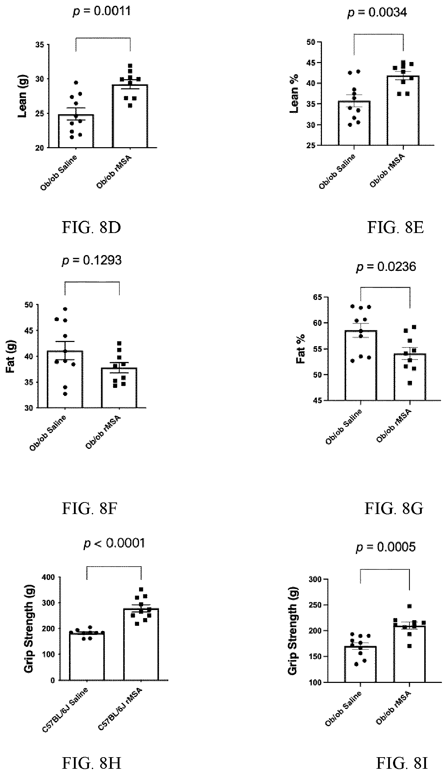

Figure 8D shows the lean mass of mice. The error bar indicates SEM.
Figure 8E shows the lean percentage of mice. The error bar indicates SEM.
Figure 8F shows the fat mass of mice. The error bar indicates SEM.
Figure 8G shows the fat percentage of mice. The error bar indicates SEM.
Figure 8H shows the values of grip strength of wild type mice. The error bar indicates SEM.
Figure 8I shows the values of grip strength of obese mice. The error bar indicates SEM.

### Detailed Description of the Invention

As used herein, the term "Human Serum Albumin" or "HSA" has the meaning well known in the art. It has the CAS NO. 70024-90-7 and a molecular weight of 66 kD.

The term "Human Serum Albumin" or "HSA" comprises the wild type human serum albumin (see, e.g., Gene name: ALB; NCBI ID: 213; UniProtKB-P02768) and any functional mutant or modification thereof. Those skilled in the art understand that certain modifications, for example, addition, deletion or substitution of one or several amino acid residue(s), can be made to the wild type HSA without substantially affecting its biological functions. Thus, the term "human serum albumin" as used herein also encompasses such modified HSA mutants or modifications.

To prepare the HSA, a variety of methods can be used. The HSAs commonly seen on the commercial market are often prepared from human blood. In addition, HSAs can also be prepared using a recombinant method. HSA products prepared by different processes can have certain differences in performance, including, for example, oxidation degree, glycation degree, and biological activity.

### Young and undamaged HSA

As used herein, the term "young and undamaged human serum albumin" or "young and undamaged HSA" refers to the HSA which is in a fresh status and has no significant damage. The "damage" here comprises, for example, oxidation, glycation, and carbonylation etc.

The skilled artisan will understand that by using the term "young and undamaged" HSA, it is not strictly required that the HSA has absolutely no damage. It is very hard to prepare an HSA without any damage. For the purpose of the present invention, an HSA comprising limited damage is still acceptable and can still be referred to as a young and undamaged HSA.

The "young and undamaged" HSA generally exhibits at least one, for example, two, three, or preferably four, of the following properties: (1) a higher ratio of free thiol in Cys-34 residue, (2) a lower level of advanced glycation end-product (AGE), (3) a lower level of carbonylation, and (4) a lower level of homocysteinylation. The young and undamaged HSA generally has a higher ratio of free thiol and lower level of AGE, carbonyl and homocysteine.

In some embodiments, the "young and undamaged" HSA exhibits at least one, for example, two, three, or preferably four, of the following properties: (1) the ratio of free thiol in Cys-34 residue is greater than 50%, for example, 70%, especially 80%, preferably 90%, and more preferably greater than 95%, as determined by the Ellman's method; (2) the level of advanced glycation end-product (AGE) is lower than 60 µg/g protein, preferably lower than 40 µg/g protein, and more preferably lower than 30 µg/g protein, as determined by ELISA (CLOUD-CLONE Co., CEB353Ge); (3) the level of carbonyl is lower than 1.7 nmol/mg protein, and preferably lower than 1.5 nmol/mg protein, as determined with the Protein Carbonyl Content Assay Kit; and (4) the level of homocysteine is lower than 5 nmol/g protein, preferably 3.5 nmol/g protein, and more preferably lower than 2 nmol/g protein, as determined by ELISA (Jianglai, JL 10022).

In some embodiments, the present invention provides a preparation of young and undamaged HSA, wherein the young and undamaged HSA exhibits at least one, for example, two, three, or preferably four, of the following properties: (1) a higher ratio of free thiol in Cys-34 residue, (2) a lower level of AGE, (3) a lower level of carbonylation, and (4) a lower level of homocysteinylation, as compared to an endogenous HSA preparation prepared from the serum of a young individual.

The term "young individual" generally refers to a person under the age of 30, preferably under the age of 18, more preferably under the age of 3, or even under the age of 1.

The term "preparation" refers to a material prepared by a process. For example, an HSA preparation may refer to an HSA sample obtained from a particular individual or from multiple individuals, which contains a population of HSA molecules. A "preparation" can be a packaged commercial product or a crude product prepared in the form of a solution or a lyophilized powder.

**Free thiol:** In a wild type HSA, there is only one free thiol group, which is located in the Cys-34 residue. Those skilled in the art can easily understand that the functional mutant of HSA may contain a slightly different amino acid sequence compared with the wild type HSA, and the Cys-34 residue may be located at a position slightly different from position 34. This can be easily determined with methods such as homology comparison.

In some embodiments, the ratio of free thiol in Cys-34 residue is determined by the Ellman's method. Preferably, the ratio of free thiol is greater than 70%, especially 80%, preferably 90%, and more preferably greater than 95%.

**AGE:** Serum albumin is a plasma protein that is highly sensitive to glycation. The glycation, also known as the Maillard reaction, is a slow, non-enzymatic reaction. Initially, it involves linking glucose or its derivatives to a free amine group of albumin to form reversibly a Schiff base product, which leads to the formation of a stable fructosamine residue (ketoamine) after the Amadori rearrangement. Then, the Amadori product can cyclize to form pyranose or furanose adducts. Further modifications to these early glycation products, such as rearrangement, oxidation, polymerization, and cleavage, give rise to irreversible conjugates called advanced glycation end-products (AGEs). The mass concentration of the AGEs can be measured with an enzyme-linked immunosorbent assay (ELISA) kit according to the manufacturer's instructions (CLOUD-CLONE Co., CEB353Ge). The results are normalized by HSA concentration per reaction and expressed in microgram AGE per gram protein.

In some embodiments, the level of AGE is determined by ELISA (CLOUD-CLONE Co., CEB353Ge). Preferably, the level of AGE is lower than 60 µg/g protein, preferably lower than 40 µg/g protein, and more preferably lower than 30 µg/g protein.

**Carbonyl:** Carbonylation here refers to the formation of carbonyl on an amino acid residue of HSA. The Protein Carbonyl Content Assay Kit (ab 126287) can be used to quantify the carbonyl in an HSA. This method is based on the reaction of DNPH with the carbonyl in the protein to form DNP hydrazones, which can be quantified through absorbance at 375 nm using a microplate reader. The molarity of carbonyl can be determined using the Protein Carbonyl Content Assay Kit (Abcam, ab 126287) according to the manual. The results are normalized by HSA concentration per reaction and expressed in nanomole carbonyl per milligram protein.

In some embodiments, the level of carbonyl is determined by the Protein Carbonyl Content Assay Kit. Preferably, the level of carbonyl is lower than 1.7 nmol/mg protein, and preferably lower than 1.5 nmol/mg protein.

**Homocysteine (HCY):** Homocysteine (HCY), which is derived from metabolism of the essential amino acid Met, is found to participate in post-translational modification (PTM) of proteins. The HCY can be linked to a protein via an isopeptide bond with a lysine (Lys) residue (N-Hcy-protein) or via a disulfide bond with a Cys-34 residue (S-Hcy-protein). N-homocysteinylation is an important PTM that affects the structure and functions of a protein, causing damage to the protein. N-homocysteinylation can be determined via the HCY-thiolactone that arises only from HCY The molarity of HCY can be quantitatively determined by ELISA or HPLC. In our study, the molarity of HCY is determined by ELISA according to the manufacturer's instructions (Jianglai, JL10022). The results are normalized by HSA concentration per reaction and expressed in nmol HCY per gram protein.

In some embodiments, the level of homocysteine is determined by ELISA (Jianglai, JL10022). Preferably, the level of homocysteine is lower than 5 nmol/g protein, preferably lower than 3.5 nmol/g protein, and more preferably lower than 2 nmol/g protein.

Those skilled in the art will understand that the four parameters above may be determined by other recognized methods, and that the numerical results of the parameters determined by different methods may vary. Those skilled in the art can easily compare the results obtained from different methods through limited experiments.

In a preferred embodiment, the ratio of free thiol in Cys-34 residue is greater than 80%, the level of AGE is lower than 30 µg/g protein, the level of carbonyl is lower than 1.5 nmol/mg protein, and the level of homocysteine is lower than 2 nmol/g protein.

The preparation of the young and undamaged HSA can be produced recombinantly or purified from human plasma. Preferably, the HSA preparation is produced recombinantly.

In order to avoid immune reactions, rMSA (recombinant mouse serum albumin) is used in the animal experiments in this study. The rMSA used in this study is young and undamaged compared with the endogenous MSAs from mice of different ages in terms of the four parameters. Both the rMSA and the rHSA used in this study are from Protgen Ltd. and are produced recombinantly.

### Pharmaceutical Composition

In another aspect, the present invention provides a pharmaceutical composition comprising the young and undamaged HSA described herein and a pharmaceutically acceptable carrier.

As used herein, the term "pharmaceutically acceptable carrier" refers to a substance which is safe for administration, for example, a solid or liquid diluent, filler, antioxidant, and stabilizer etc. Depending on the route of administration, various carriers well known in the art may be administered, including but not limited to carbohydrate, starch, cellulose and derivatives thereof, maltose, gelatin, talc, calcium sulfate, vegetable oil, synthetic oil, polyol, alginic acid, phosphate buffer, emulsifier, isotonic saline, and/or pyrogen-free water.

The "treat", "treatment" or "treating" herein comprises, for example, cure, alleviation of a symptom, and prolongation of survival.

As used herein, the term "therapeutically effective amount" refers to an amount of an active substance sufficient to elicit a biological or medical response expected by a clinician in a subject. The "therapeutically effective amount" can be determined by those skilled in the art based on the administration route, the subject's body weight, age, and condition, and other factors. For example, a typical daily dose may range from 0.01 mg to 100 mg active ingredient per kg body weight.

The medicament provided by the present invention can be prepared into clinically acceptable dosage form such as injection. The pharmaceutical composition of the present invention can be administered to a subject in any suitable route, for example, administered by intravenous infusion.

The term "subject" encompasses mammals, including human subject in need of treatment as well as non-human mammals such as primate, cow, sheep, cat, and dog. The subject may also be a healthy person.

Unless otherwise stated, scientific and technical terms used in this specification have the meaning commonly understood by those of ordinary skill in the art. Generally, the nomenclatures and techniques used in this specification in relation to cell and tissue culture, molecular biology, genetics, and protein and nucleic acid chemistry are well known and commonly used in the art.

In order to explain the present invention in more detail, examples of the present invention are provided below with reference to the accompanying drawings. These examples are for purpose of illustration and description only and should not be construed as limiting the scope of the present invention.

### Example 1: Injection of the young and undamaged recombinant albumin allowed the blood glucose level to return to normal in human and in mice

Diabetes mellitus refers to a group of lifelong metabolic diseases characterized by chronic hyperglycemia and can be induced by various causes. Due to a high level of blood glucose in a long term, great vessels and capillaries can be damaged and the heart, brain, kidney, peripheral nerve, eye, foot and the like may be endangered. According to the statistics from the World Health Organization, diabetes has more than 100 complications, the largest number of complications currently known for a disease. More than half of the deaths from diabetes are caused by cardiovascular and cerebrovascular diseases, and 10% are caused by nephropathy. The number of patients having amputations due to diabetes is 10-20 times that of patients having amputations not due to diabetes. Clinical data show that about 10 years after the onset of diabetes, 30-40 % of the patients have at least one complication, and once a complication occurs, it can be hardly reversed by treatment with a medicament. Thus, early prevention of diabetes complications is emphasized.

Normally, the balance between the generation and the removal of blood glucose in the body is maintained mainly by the hormone and the nerve system regulating and keeping the blood glucose at a certain level. However, a genetic factor (e.g. a family history of diabetes) combined with an environmental factor (e.g. an unhealthy diet or obesity) can disturb the function of the two regulatory systems, leading to increase of the blood glucose.

A high level of blood glucose in a long term will cause lesion in various tissues and organs of the body, giving rise to an acute or chronic complication. Such a complication can be, for example, dehydration, electrolyte imbalance, nutritional deficiency, weakened immunity, impaired renal function, neuropathy, fundus lesion, cardiovascular and cerebrovascular disease, and diabetic feet. It is therefore necessary to control the blood glucose level.

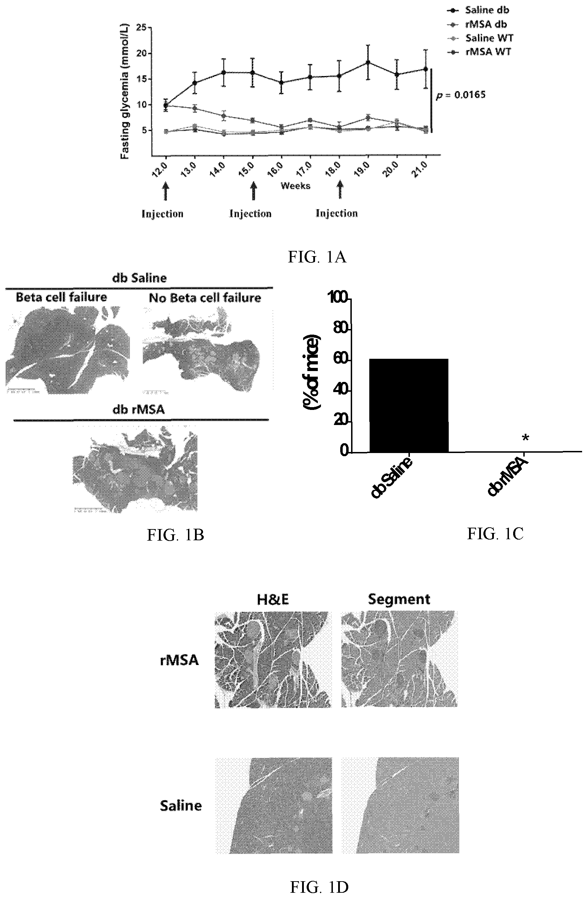

In order to investigate whether the young and undamaged rHSA has the efficacy of lowering the blood glucose level, we injected the young and undamaged rMSA or normal saline as a control to the tail vein of the mice with type II diabetes (db/db) and the wild type mice (WT) every three weeks, and monitored the fasting glycemia of the mice every week. It can be seen from the results that the rMSA effectively lowered the fasting glycemia level in mice with type II diabetes after three injections (FIG. 1A). Thereafter, H&E staining of pancreas sections was carried out. We found that many mice in the group with saline injections had a smaller islet size and beta cell failure, while the group with rMSA (Protgen) injections did not show such changes (FIGs. 1B and 1C). We also found that for the db mice, the islet percentage and the relative islet size of the rMSA-treated group were significantly larger than those of the saline group (FIGs. 1D-F). Thereafter, we carried out TUNEL staining on sections of mouse pancreas and analyzed the islet beta cell death. We found that TUNEL signal-positive beta cells were significantly less in the rMSA-treated group than in the saline-treated group. This indicated that more islet beta cells died in the saline group (FIGs. 1G and 1H). In addition, we examined the CC3 level in sections of db mouse islets to determine the apoptosis level of islet beta cells. The results showed that more islet beta cells underwent apoptosis in the normal saline-treated group than in the rMSA-treated group of db mice (FIGs. 1I and 1J).

Further, to confirm the effect of the rMSA in delaying type II diabetes, we carried out an intraperitoneal glucose tolerance test (IPGTT). The IPGTT results showed that the db mice that received two injections of the rMSA exhibited better glucose tolerance than those in the control group which received physiological saline (FIGs. 1K and 1L). In addition, the body composition of db mice was determined by EcoMRI after two injections. It was found that the db mice injected with the rMSA had a significantly decreased fat percentage and a significantly increased lean percentage compared with the db mice injected with physiological saline (FIGs. 1M and 1N).

To continue the investigation about whether the young and undamaged rHSA has the efficacy of lowering the blood glucose level, we recruited a patient with type II diabetes, injected the young and undamaged rHSA to the patient intravenously, and monitored the fasting glycemia of the patient every week. It can be seen from the results that the rHSA effectively lowered the fasting glycemia level in the patient with type II diabetes (FIG. 1O).

### Example 2: The preparation of the young and undamaged rHSA was able to bind free fatty acids and contained less free fatty acids than the commercially available preparation of blood-derived HSA

An elevated level of plasma free fatty acid (FFA) may be one of the important factors for insulin resistance in obese patients with type II diabetes.

In order to determine the ability of the preparation of the young and undamaged rHSA to bind FFAs, we mixed the albumin with the FFAs, and then obtained the absorbance at 360 nm. The results showed that the rHSA (Protgen) preparation was able to bind the 683 µM FFAs (palmitic acid : stearic acid : oleic acid : linoleic acid = 27 : 10 : 32 : 15; the concentration of FFAs and the ratio of the various types of fatty acids were consistent with those in the serum of a healthy person ^{[1]}) (FIG. 2A).

We used the Free Fatty Acid (FFA) Content Assay Kit (Boxbio, AKFA008M) to determine the amount of FFA carried by the two HSAs. The FFA could bind to a copper ion to form fatty acid-copper, which was then dissolved in chloroform. Then, the copper ion therein reacted with a color developing solution to form a purple complex, which showed a characteristic absorption peak at 550 nm. By obtaining the change of the absorbance, the FFA content was determined quantitatively. The results showed that the rHSA (Protgen) preparation itself contained a lower amount of FFA than the commercially available preparation of blood-derived HSA (FIG. 2B).

### Example 3: The young and undamaged rHSA was able to bind cholesterol and had a stronger structural stability than the commercially available blood-derived HSAs

The lipid metabolism disorder forms a basis for the development of atherosclerosis, wherein cholesterol is the main component of the plaque. The accumulation of cholesterol under the vascular endothelium continues throughout the whole process of atherosclerosis. Clinical studies have shown that a higher plasma cholesterol level is associated with a greater risk of a cardiovascular or cerebrovascular disease. Thus, the following experiments were designed and carried out in order to investigate whether the rHSA has the ability to bind cholesterol and reduce the accumulation of cholesterol.
1. In an aqueous solution, the same concentration (30 µM) of the rHSA (Protgen) was allowed to react with a varying concentration (0, 30, and 60 µM) of cholesterol respectively in a water bath at 37°C for 10 min. After cooling to room temperature and exciting at 295 nm, the mixture was scanned at 300-400 nm for the fluorescence emission spectrum (FIG. 3A).
2. In an aqueous solution, the same concentration (30 µM) of any one of two commercially available blood-derived HSAs was allowed to react with a varying concentration (0, 30, and 60 µM) of the cholesterol respectively in a water bath at 37°C for 10 min. After cooling to room temperature and exciting at 295 nm, the mixture was scanned at 300-400 nm for the fluorescence emission spectrum (FIG. 3B-C).

The results of the experiments were analyzed as follows:
1. According to FIG. 3A, as the cholesterol concentration increased, the fluorescence emission intensity of the rHSA gradually decreased, and the excitation light intensity of Trp214 was inhibited, demonstrating that cholesterol was bound to the rHSA molecule.
2. According to FIG. 3B, as the cholesterol concentration increased, the emission fluorescence intensity of the first commercially available blood-derived human serum albumin (HSA-1) firstly increased and then decreased. This demonstrated that the HSA-1 was not tightly bound to the cholesterol at a low concentration (red line), and thus the HSA-1 had a weaker ability to bind cholesterol than the rHSA. According to FIG. 3C, as the cholesterol concentration increased, the fluorescence emission intensity of the second commercially available blood-derived human serum albumin (HSA-2) gradually decreased, but the curve fluctuated significantly at a high concentration (blue line), demonstrating that the HSA-2 had a weaker ability to bind cholesterol than the rHSA.
3. According to FIGs. 3A-C, the maximum fluorescence emission intensity appeared at 338 nm for the rHSA, which was consistent with the report in the literature ^{[2-3]}. And it appeared at 350 nm, which is significantly greater than 338 nm, for the two commercially available blood-derived HSAs, demonstrating that their molecular structure was looser and less stable.

Based on the above experimental results, the following conclusions could be drawn:
1. The rHSA had the ability to bind cholesterol.
2. The rHSA had a stronger ability to bind cholesterol than the commercially available blood-derived HSAs.
3. The rHSA had a more stable structure than the commercially available blood-derived HSAs.

### Example 4: Injection of the young and undamaged rHSA could treat Alzheimer's disease

Alzheimer's disease is one of the most common neurodegenerative diseases that cause senile dementia, and its incidence increases dramatically with age. As the global aging intensifies, Alzheimer's disease has become a more common cause of death and cannot be prevented, cured, or even slowed down. The currently most mainstream hypothesis on the pathogenesis of Alzheimer's disease, the Aβ cascade hypothesis, believes that the Alzheimer's disease starts with the abnormal accumulation of Aβ, which leads to a series of cascade reactions resulting in neurofibrillary tangles, synaptic loss and neuronal death and the like.

Albumin is the most abundant protein in human serum. In addition to maintaining the plasma colloid osmotic pressure, albumin can also bind and transport many substances in the blood, such as free fatty acid, metal ion and Aβ peptide.

To demonstrate that intravenous injection of the young and undamaged rHSA could treat Alzheimer's disease, we compared the young and undamaged rHSA and the blood-derived HSA for their ability to inhibit the aggregation of Aβ peptides *in vitro* by thioflavin staining. Thioflavin is a small-molecule compound of benzothiazole type. It can specifically bind to amyloid fibrils, and give a strong emission wave which was detectable at about 485 nm under the irradiation of excitation light at about 450 nm. The strength of the fluorescence signal provided by the emission wave is in proportion to the amount of the amyloid fibrils.

The results (FIG. 4) showed that when 10 mg/ml of Aβ peptides, and 50 mg/ml of the rHSA or 50 mg/ml of the blood-derived HSA were used, the rHSA showed a significantly stronger ability to inhibit the aggregation of Aβ peptides than the blood-derived HSA at every time point in the experiment. The results at 0 h showed that the preparation of the commercially available blood-derived HSA itself contained amyloid fibrils having a content 6.16 times that of the rHSA preparation (Protgen) (p < 0.0001). At 24 h, the rHSA preparation (Protgen) showed an ability to inhibit the aggregation of Aβ peptides 6.12 times that of the commercially available blood-derived HSA (p = 0.0055).

Based on these results, we believed that the albumin played an important role in the progression of Alzheimer's disease. Subsequently, we established an Alb-/-, 5×FAD mouse using the existing Alb knockout mouse model and the 5×FAD transgenic mouse model of Alzheimer's disease through hybridization. In the Y-maze experiment, mice were placed into an acrylic maze having a length of 30 cm, a width of 15 cm, a height of 15 cm, and an angle of 120° between the three arms. The mice were allowed to explore the maze freely for 8 min, and the number of entry of the mouse into each arm was recorded. If a mouse entered a different arm for three consecutive times, it was recorded as "right" for one time. Based on this definition, the right ratio was calculated to evaluate the spatial and the immediate memory levels of the mice. As shown in FIG. 4B, the 3-month-old Alb-/-, 5×FAD mice performed significantly worse in the Y-maze than their littermates, the Alb+/-, 5×FAD mice and the Alb+/- mice. This demonstrated that the albumin had the effect of inhibiting the progression of Alzheimer's disease at the early stage.

### Example 5: Injection of the young and undamaged recombinant albumin could delay the Parkinson's disease

Parkinson's disease (PD) is a common neurodegenerative disease caused by the degeneration and death of dopaminergic neurons in the substantia nigra in the midbrain, resulting in a significant decrease of the content of striatal dopamine (DA). Clinically, patients with PD mainly show resting tremor, bradykinesia, muscle rigidity, and postural and gait disorders, which may be accompanied by non-motor symptoms such as depression, hyposmia, constipation and sleep disorder. According to statistics, China has nearly 3 million patients with PD, accounting for about half of the world's total number. Thus, China is the country having the greatest number of patients with PD. PD mostly occurs in the elderly over the age of 60. With about 100,000 people newly diagnosed with PD in China every year, the incidence rate of those over the age of 65 is as high as 1.7%. Usually, at the time when a person is diagnosed with PD, more than 50% of the neurons in the substantia nigra in the patient's midbrain may have died. Moreover, PD will worsen with age. Many people call PD an "immortal cancer".

The exact etiology of PD is still unknown. Genetic and environmental factors, aging, oxidative stress and other factors may be involved in the process of degeneration and death of dopaminergic neurons in the substantia nigra. Current treatments for PD include drug therapy and surgery, both of which can only relieve symptoms, but cannot cure or halt the progression of the disease.

In recent years, an increasing number of studies have shown that in patients with PD, the level of oxidative stress in the nervous system is significantly increased, and the level of reactive oxygen species (ROS) in the neurons in the substantia nigra is significantly higher, which may be a major factor resulting in the further development of the disease in patients with PD.

In order to investigate whether the young and undamaged rHSA can prevent ROS from damaging nerve cells, we induced neurotoxicity in the human neuroblastoma cells (SH-SY5Y) using the 1-methyl-4-phenylpyridine ion (MPP+), simulating the process of damaging neurons in the substantia nigra in a PD patient at a cellular level. Then, the SH-SY5Y cells with neurotoxicity induced by MPP+ were incubated with the young and undamaged rHSA or the commercially available blood-derived HSA respectively.

As shown in FIG. 5A, the SH-SY5Y cells were cultured to a density of 70-80% and the MPP+ (0.5 mM) was added. Immediately after the addition, different concentrations of the rHSA or the commercially available blood-derived HSA were added. After treatment under normal cell culture conditions for 24 h, the ROS level in cells was determined using DCFH-DA (10 µM, with treatment in an incubator at 37°C for 30 min). The results showed that both the young and undamaged rHSA and the commercially available blood-derived HSA significantly reduced the ROS level in the SH-SY5Y cells. And the young and undamaged rHSA showed a significantly better effect of reducing the cellular ROS level than the commercially available HSA at the same concentration.

In the experiment using an animal model, we induced PD-like symptoms in mice using MPTP, and then injected the rMSA to the mice intravenously. By testing the grip strength of mice, we found that the exogenous rMSA increased the grip strength of all the mice having PD-like symptoms, regardless of the severity of the symptoms induced by the different doses of MPTP (FIGs. 5B and 5C).

The behavioral tests usually adopted to test the motor functions of mice mainly include the beam traversal test and the pole descend test. From the results of the tests, we saw that the exogenous rMSA enhanced the motor functions of mice having PD-like symptoms induced by MPTP (FIGs. 5D-G).

The experimental methods and materials were as follows:
Reactive oxygen species (ROS) test: The SH-SY5Y cells were cultured with the RPMI1640 medium containing 10% FBS and 1% streptomycin/penicillin at 37°C with 5% CO₂. The medium was replaced every 2-3 days. The groups included in the experiment were as follows: a control group (untreated), a PD model group (induced with 0.5 mM MPP+), a group with MPP+ induction and commercial HSA treatment (5, 10 or 20 mg/mL), and a group with MPP+ induction and rHSA treatment (5, 10 or 20 mg/mL). The level of ROS in cells was determined using DCFH-DA as a fluorescence probe. The cells that were subjected to MPP+ induction and treated with rHSA for 24 h were washed three times with serum-free medium and treated with 10 µmol/L DCFH-DA at 37°C for 30 min, followed by washing twice with serum-free medium. Subsequently, the fluorescence from DCF was detected using a microplate reader, with an excitation wavelength of 485 nm and an emission wavelength of 538 nm. The results were expressed as mean ± standard error for three replicates.

Treatment of animal: Adult male C57BL/6J mice (3-month-old, 25-30 g) were housed in mouse cages with 12 h light per day and had free access to food and water. Two batches of mice were used and each batch was divided into three groups (4-8 mice per group). The three groups in a first batch were injected with physiological saline (0.9% NaCl) and/or MPTP (20 mg/kg/day) intraperitoneally for 7 consecutive days respectively. The three groups in a second batch were injected with physiological saline (0.9% NaCl) and/or MPTP (30 mg/kg/day) intraperitoneally for 7 consecutive days respectively. After one week from the intraperitoneal injections, the mice were intravenously injected with physiological saline (0.9% NaCl) or rMSA (1.5 mg per gram of mouse) every 3 weeks. After 8 intravenous injections, the mice were subjected to behavioral tests, including the grip strength test, the beam traversal test and the pole descend test.

Grip Strength Test: A grip strength meter for mice and rats was used to perform the test. A mouse was allowed to grab the metal grid, and then its tail was gently pulled backward at a constant speed until the mouse can no longer grasp the grid. The test was repeated five times for each mouse, and the obtained values were averaged. The test was a randomized, double-blind test.

Beam Traversal Test: The 1-meter-long beam of organic glass was made to include four sections, wherein each section had an equal length of 0.25 m and a width of 3.5 cm, 2.5 cm, 1.5 cm or 0.5 cm. The widest part of the beam was attached to the mouse loading platform and the narrowest part was attached to the cage. Animals were trained to traverse a beam for two days prior to the test. During each day of training, the animals underwent 3-5 trials so that they could move forward and traverse the beam without any assistance. On the third day, a formal test was performed to record the time for the mice to traverse the beam from the loading platform to the cage. For each animal, the test was repeated three times and the results were averaged. Timing was started when the animal's forelimb reached the 2.5 cm-wide section and ended when a forelimb reached the cage.

Pole Descend Test: A pole having a length of 0.5 m and a diameter of 1 cm was placed in a cage. The surface of the pole was wrapped with an anti-slip pad to facilitate grasping by the animals. Animals were trained for two days before the test. On the first day of training, animals underwent 3 trials. They were placed, with their heads downwards, at a height of 1/3 of the pole length above the bottom of the cage in the first trial, at a height of 2/3 of the pole length in the second trial, and on top of the pole in the third trial, and allowed to descend to the bottom of the cage. On the second day of training, the animals underwent three trials of descending from the top of the pole. On the test day, the animals were placed, with their heads downwards, on top of the pole and the time for them to descend to the bottom of the cage was recorded. For each animal, the test was repeated three times and the results were averaged. Timing was started when the animal was released on top of the pole by the operator and ended when the animal reached the bottom of the cage with one of its hindlimb.

### Example 6: The young and undamaged rHSA were different from the commercially available blood-derived HSAs in four age-related indicators

In order to distinguish the young and undamaged rHSA from the blood-derived HSAs, we purchased 3 HSA products from different manufacturers, and made a comparison in terms of the free thiol content, the carbonylation level, the AGE level and the homocysteine modification level. The results were as follows.

Firstly, we used Ellman's method to detect the absolute content of free thiol, and then divided it by the amount of HSA (theoretically, one HSA molecule only contained one free thiol) to calculate the percentage content of residual free thiol. The results showed that the rHSA (Protgen) contained intact free thiol, whereas the free thiol in the blood-derived HSAs had been seriously destroyed (102.8% v.s. 17.6%, p < 0.0001) (FIG. 6A).

Secondly, we used the Protein Carbonyl Content Assay Kit (Abcam, ab126287) to detect the molarity of carbonyl according to the instructions. The results were normalized by HSA concentration per reaction and expressed in nmol carbonyl per mg protein (nmol/mg protein). The results showed that the carbonyl level of the rHSA (Protgen) was lower than that of the blood-derived HSAs (1.46 v.s. 1.80 nmol/mg protein, p = 0.0072) (FIG. 6B).

Thirdly, we measured the mass concentration of the advanced glycation end-product (AGE) using an ELISA kit (CLOUD-CLONE Co., CEB353Ge) according to the instructions. The results were normalized by HSA concentration per reaction and expressed in µg AGE per mg protein (µg/mg protein). The results showed that the AGE level of the rHSA (Protgen) was significantly lower than that of the blood-derived HSAs (21.4 v.s. 66.8 µg/g protein, p < 0.0001) (FIG. 6C).

Fourthly, we measured the molarity of homocysteine (HCY) using the ELISA kit (Jianglai, JL10022) according to the instructions. The results were normalized by HSA concentration per reaction and expressed in nmol HCY per gram protein (nmol/g protein). The results showed that the HCY level of the rHSA (Protgen) was much lower than that of the blood-derived HSAs (1.46 v.s. 5.56 nmol/g protein, p = 0.0063) (FIG. 6D).

Comparison of the four indicators between the rHSA (Protgen) and the three commercially available blood-derived HSAs was provided in Table 1.

### Example 7: The young and undamaged recombinant albumin lowered the blood cholesterol level and the risk of atherosclerosis

The total blood cholesterol and the low-density lipoprotein cholesterol (LDL-C) in patients with hypoalbuminemia were significantly higher than those in healthy people. The albumin knockout (AKO) mice, having been fed with a Western diet, showed significantly higher total blood cholesterol and LDL-C than the wild type (WT) mice. And supplementation of the rMSA to the AKO mice significantly reduced the LDL-C level (FIG. 7). The control group was injected with the same volume of physiological saline. WT mice were used as baseline controls. The supplementation was performed through the tail vein injection. For the cycle and the dose, injections at a dose of 1.5 g/kg body weight were given at Week 1, 2, and 4 from the initiation of the experiment.

The experimental methods and materials were as follows:
Mouse model: Albumin knockout (AKO) mice were purchased from The Jackson Laboratory (Cat. No. 025200). During normal feeding days, the mice had free access to water and food. The house environment was maintained with 12 h light/12 h dark. The temperature was 23-25°C.

Feeds: The Western diet was purchased from Research diet. (Cat. No. D12079B) and included 40% kcal fat and 0.15% cholesterol. The normal diet was purchased from Jiangsu Xietong Pharmaceutical Bio-engineering (Cat. No. SWC9102) and included 12% kcal fat and no cholesterol.

Blood lipid determination: Starvation was carried out for 12 h. Blood samples were collected from the tail tips of mice, placed at room temperature for 2 h for coagulation, and then centrifuged at 3000 rpm for 15 min at 4°C to obtain the serum. The total cholesterol and the LDL-C in the serum were determined with the kits purchased from Nanjing Jiancheng (Cat. Nos. A111-1-1 and A113-1-1).

### Example 8: The young and undamaged recombinant albumin could treat obesity

Obesity refers to a certain degree of obvious overweight and an excessively thick fat layer. It is a state caused by excessive accumulation of lipids, especially the triglycerides, in the body. It is not directed to simply a weight gain, but the excessive accumulation of the adipose tissue in the body. Due to excessive food intake or changes of the metabolism in the body, an excessive amount of lipids accumulates in the body, resulting in an excessive weight gain, and pathological or physiological changes or a tendency of such changes in the human body. Obesity not only affects the physical beauty, but also brings inconvenience to life. It may also lead to joint and soft tissue injury, reduced reproductive capacity, psychological disorder, heart disease, diabetes, atherosclerosis, fatty liver, gallstones, edema, and gout, and the like.

In order to determine the effect of the rMSA in treating obesity, we used the obese model, the ob mouse. We monitored respiration in the rMSA-injected mice and the saline-injected mice using the TSE metabolic cage system. The results showed that the rMSA significantly increased the oxygen consumption, carbon dioxide production and respiratory exchange ratio (RER) in ob mice (FIGs. 8A-C).

We measured the body composition of the mice with the Echo MRI-100 instrument, focusing on muscle and fat contents. The results showed that the rMSA-treated mice had a greater muscle content and a lower fat content than the saline-treated mice (FIGs. 8D-G). We also tested the grip strength of the mice using the grip strength metering system, and found that the rMSA-treated mice had stronger grip strength than the saline-treated mice (FIGs. 8H-I).

**Table 1. Comparison of four indicators between the rHSA and the blood-derived HSAs from different manufacturers**

| **Indicator** | **Protgen** | **Product 1** | **Product 2** | **Product 3** | **Mean of the three products** |
|---|---|---|---|---|---|
| **Free thiols (%)** | 102.8 | 9.65 | 31.7 | 11.3 | 17.6 |
| **Carbonyl (nmol/mg protein)** | 1.46 | 1.77 | 1.71 | 1.91 | 1.80 |
| **AGE (µg/g protein)** | 21.4 | 62.5 | 71.8 | 66.1 | 66.8 |
| **HCY (nmol/g protein)** | 1.46 | 5.35 | 6.34 | 5.00 | 5.56 |

### REFERENCE

[1] Finn T E, Nunez A C, Sunde M, et al. Serum Albumin Prevents Protein Aggregation and Amyloid Formation and Retains Chaperone-like Activity in the Presence of Physiological Ligands[J]. Journal of Biological Chemistry, 2012, 287(25):21530-40.
[2] Peng W, Ding F, Peng Y K. In vitro evaluation of the conjugations of neonicotinoids with transport protein: photochemistry, ligand docking and molecular dynamics studies[J]. RSC advances, 2016, 6(3): 1826-1843.
[3] Peng L, Minbo H, Fang C, et al. The interaction between cholesterol and human serum albumin[J]. Protein and peptide letters, 2008, 15(4): 360-364.

## Claims

1. Use of human serum albumin (HSA) in the manufacture of a medicament for treating a disease selected from the group of diabetes mellitus, obesity, atherosclerosis, Alzheimer's disease, and Parkinson's disease.

2. The use of claim 1, wherein the HSA is an HSA prepared from human blood.

3. The use of claim 1, wherein the HSA is a young and undamaged HSA.

4. The use of claim 3, wherein the young and undamaged HSA exhibits at least one, for example, two, three, or preferably four, of the following properties: (1) a higher ratio of free thiol in Cys-34 residue, (2) a lower level of advanced glycation end-product (AGE), (3) a lower level of carbonylation, and (4) a lower level of homocysteinylation, as compared to an endogenous HSA preparation prepared from the serum of a young individual.

5. The use of claim 3, wherein the young and undamaged HSA exhibits at least one, for example, two, three, or preferably four, of the following properties: (1) the ratio of free thiol in Cys-34 residue is greater than 50%, for example, 70%, especially 80%, preferably 90%, and more preferably greater than 95%, as determined by the Ellman's method; (2) the level of advanced glycation end-product (AGE) is lower than 60 µg/g protein, preferably lower than 40 µg/g protein, and more preferably lower than 30 µg/g protein, as determined by ELISA (CLOUD-CLONE Co., CEB353Ge); (3) the level of carbonyl is lower than 1.7 nmol/mg protein, and preferably lower than 1.5 nmol/mg protein, as determined by the Protein Carbonyl Content Assay Kit; and (4) the level of homocysteine is lower than 5 nmol/g protein, preferably lower than 3.5 nmol/g protein, and more preferably lower than 2 nmol/g protein, as determined by ELISA (Jianglai, JL10022).

6. The use of any one of claims 3-5, wherein the ratio of free thiol in Cys-34 residue is greater than 80%.

7. The use of any one of claims 3-6, wherein the level of AGE is lower than 30 µg/g protein.

8. The use of any one of claims 3-7, wherein the level of carbonyl is lower than 1.5 nmol/mg protein.

9. The use of any one of claims 3-8, wherein the level of homocysteine is lower than 2 nmol/g protein.

10. The use of any one of claims 3-9, wherein the ratio of free thiol in Cys-34 residue is greater than 80%, the level of AGE is lower than 30 µg/g protein, the level of carbonyl is lower than 1.5 nmol/mg protein, and the level of homocysteine is lower than 2 nmol/g protein.

11. The use of any one of claims 3-10, wherein the HSA is produced recombinantly.

12. A method of treating a disease, comprising administering to a subject a therapeutically effective amount of human serum albumin (HSA), wherein the disease is selected from the group consisting of diabetes mellitus, obesity, atherosclerosis, Alzheimer's disease, and Parkinson's disease.

13. Use of human serum albumin (HSA) in treating a disease selected from the group of diabetes mellitus, obesity, atherosclerosis, Alzheimer's disease, and Parkinson's disease.

14. Use of a pharmaceutical composition comprising a human serum albumin (HSA) and a pharmaceutically acceptable carrier in treating a disease, wherein the disease is selected from the group of diabetes mellitus, obesity, atherosclerosis, Alzheimer's disease, and Parkinson's disease.

15. The method or use of any one of claims 12-14, wherein the HSA is an HSA prepared from human blood.

16. The method or use of any one of claims 12-14, wherein the HSA is a young and undamaged HSA.

17. The method or use of claim 16, wherein the young and undamaged HSA exhibits at least one, for example, two, three, or preferably four, of the following properties: (1) a higher ratio of free thiol in Cys-34 residue, (2) a lower level of advanced glycation end-product (AGE), (3) a lower level of carbonylation, and (4) a lower level of homocysteinylation, as compared to an endogenous HSA preparation prepared from the serum of a young individual.

18. The method or use of claim 16, wherein the young and undamaged HSA exhibits at least one, for example, two, three, or preferably four, of the following properties: (1) the ratio of free thiol in Cys-34 residue is greater than 50%, for example, 70%, especially 80%, preferably 90%, and more preferably greater than 95%, as determined by the Ellman's method; (2) the level of advanced glycation end-product (AGE) is lower than 60 µg/g protein, preferably lower than 40 µg/g protein, and more preferably lower than 30 µg/g protein, as determined by ELISA (CLOUD-CLONE Co., CEB353Ge); (3) the level of carbonyl is lower than 1.7 nmol/mg protein, and preferably lower than 1.5 nmol/mg protein, as determined by the Protein Carbonyl Content Assay Kit; and (4) the level of homocysteine is lower than 5 nmol/g protein, preferably lower than 3.5 nmol/g protein, and more preferably lower than 2 nmol/g protein, as determined by ELISA (Jianglai, JL10022).

19. The method or use of any one of claims 16-18, wherein the ratio of free thiol in Cys-34 residue is greater than 80%.

20. The method or use of any one of claims 16-19, wherein the level of AGE is lower than 30 µg/g protein.

21. The method or use of any one of claims 16-20, wherein the level of carbonyl is lower than 1.5 nmol/mg protein.

22. The method or use of any one of claims 16-21, wherein the level of homocysteine is lower than 2 nmol/g protein.

23. The method or use of any one of claims 16-22, wherein the ratio of free thiol in Cys-34 residue is greater than 80%, the level of AGE is lower than 30 µg/g protein, the level of carbonyl is lower than 1.5 nmol/mg protein, and the level of homocysteine is lower than 2 nmol/g protein.

24. The method or use of any one of claims 16-13, wherein the HSA is produced recombinantly.
